# EUROPEAN PATENT SPECIFICATION

(11) **EP 3 847 952 B1**
(45) Date of publication and mention of the grant of the patent: **10.08.2022**
(21) Application number: 20214445.7
(22) Date of filing: 16.12.2020
(51) Int. Cl.: A61B 5/00

(54) **BIOMECHATRONIC DEVICE FOR AUTOMATED DIAGNOSIS OF RHEUMATIC DISEASES**
BIOMECHATRONISCHE VORRICHTUNG ZUR AUTOMATISIERTEN DIAGNOSE RHEUMATISCHER ERKRANKUNGEN
DISPOSITIF BIOMÉCATRONIQUE POUR LE DIAGNOSTIC AUTOMATISÉ DES MALADIES RHUMATISMALES

(30) Priority: 17.12.2019 IT 201900024202
(43) Date of publication of application: 14.07.2021
(73) Proprietor: Università Degli Studi Magna Graecia Catanzaro, 88100 Catanzaro (IT)
(72) Inventor: MEROLA, Alessio, 88100 Catanzaro (CZ) (IT); RANDAZZINI, Luigi, 88100 Catanzaro (CZ) (IT); COSENTINO, Carlo, 88100 Catanzaro (CZ) (IT); GREMBIALE, Rosa Daniela, 88056 Tiriolo (CZ) (IT)
(74) Representative: Giuliano, Natalia

(56) References cited:
- WO-A1-2010/064200
- WO-A1-2011/117901
- WO-A1-2019/182188
- CN-U- 208 876 564
- JP-B2- 5 211 058

## Description

The present invention relates to a biomechatronic device for automated diagnosis of rheumatic diseases.

In particular, a biomechatronic device for automated diagnosis of rheumatic diseases is disclosed, of the type that includes wearable prosthetic components, able to be managed by means of fixed or portable terminals, equipped with dedicated software programs designed aimed at acquiring data and controlling, locally and remotely.

The reference technical field is the bioengineering, more specifically the biomechatronics, which combines technologies typical of the electronics and mechanical sectors, applying them to the motor functions of the human being.

Several biomechatronic devices, able to stimulate, by means of a mechanical action, either continuous or pulsed, different portions of the human body in order to assist healthcare personnel in medical diagnosis or for targeted therapeutic purposes, for example with reference to functional rehabilitation following sudden and traumatic events such as strokes, or chronic conditions such as rheumatoid arthritis, are currently known.

A first example of a known biomechatronic device is described in the international application WO2010033055A1 whose subject is an equipment for inducing controlled vibratory stimulation to individual fingers or other parts of a hand comprising vibration exciters adapted to said parts of the hand either directly or incorporated in a glove, fed with signals from an external signal source preprogrammed for delivery of defined vibration stimuli to said fingers or parts of the hand. Pulses of vibrations are delivered to individual fingers according to a pre-programmed pattern so that the vibration stimuli, applied consecutively to individual fingers, are separated spatially and in time with possibilities for variations in frequency, intensity, length of vibration pulses and intermission between vibration pulses.

Still, the international WO2019182188A1 describes a glove-type wrist joint therapy device comprising a finger support part including a base part disposed at the upper portion of each finger, a band part, which is made from an elastic material, is mounted at the lower portion of the base part, and is formed in a ring shape so as to support a body portion of the finger while being inserted around the circumference of the finger, and a thimble part, which is mounted at the lower portion of the base part and is formed in a thimble shape so as to support the tip of the finger while the first joint of the finger is inserted therein. In addition, the glove-type wrist joint therapy device includes a laterally expandable airbags disposed between the respective thimble parts, and expanding so as to laterally widen the gap between stiff fingers when air is injected therein and contracting so as to narrow the widened gap when the injected air is discharged; and an air pressure generator connected to the laterally expandable airbag by means of an air tube so as to inject the air into each laterally expandable airbag.

The utility model CN208876564U discloses a device and a system for detecting and transmitting hand grip strength parameters of a joint swelling and pain hand, so that the device can be used for acquiring hand grip strength parameter data of the joint swelling and pain hand, and transmitting the hand grip strength parameter data to the outside. A method, in addition, can be used for acquiring the hand grip strength parameter data of the patient with joint swelling and pain to obtain a hand grip strength-time change curve. The maximum grip strength evaluation value of the hand is uploaded to a network server, which carries out recording processing and issues the information to the terminal of a doctor. Other relevant prior art is disclosed in WO 2019/182188 A1, WO 2011/117901 A1, JP 5211058 B2.

Finally, the utility model CN108392375A describes a pneumatic soft-body function rehabilitation glove which can provide help for the hand exercise rehabilitation of stroke patients, preoperative patients, postoperative patients and the like. The pneumatic soft-body function rehabilitation glove comprises a body, an air pressure sensor, speed and acceleration sensors, an arithmetic unit, a detachable battery, a one-way bending layer, an electromagnetic valve, a miniature air pump and elastic bodies, wherein the glove body mainly comprises a glove outer surface layer and a glove inner surface layer which are used for wrapping devices such as the elastic bodies, air tubes and air bags inside the glove body.

However, the known biomechatronic devices, such as those described, suffer from shortcomings regarding the impossibility of automating medical procedures, like the measurement of the pain threshold of users/patients suffering from pathologies, or the same measurement related to healthy users, as well as of evaluating, automatically, an index of severity of the same pathologies, for example the rheumatoid arthritis.

The purpose of the present invention is to provide a biomechatronic device for automated diagnosis of rheumatic diseases that allows to supply to healthcare personnel an effective and non-invasive support for the diagnostic and therapeutic practice of the aforementioned pathologies, standardizing and automating the measurement of the pain threshold originating from mechanical nociceptive stimuli, having, therefore, characteristics such as to overcome the limits that still affect the current biomechatronic devices, with reference to the known technique.

Another purpose of the present invention is to make the measurement of the pain threshold objective, regardless of the specific physician or operator who performs the diagnostic test.

A further purpose of the present invention is to provide a biomechatronic device for automated diagnosis of rheumatic diseases which allows to automate the evaluation of the severity of the diseases.

According to the la present invention, a biomechatronic device for automated diagnosis of rheumatic diseases is provided, as defined in claim 1.

For a better understanding of the present invention, a preferred embodiment is now described, purely by way of nonlimiting example, with reference to the attached drawings, in which:
- figure 1 shows a block diagram of the architecture of a biomechatronic device for automated diagnosis of rheumatic diseases, according to the invention;
- figure 2 shows an overall view of a wearable component of the biomechatronic device for automated diagnosis of rheumatic diseases able to applied to a hand and to a wrist of a user, according to the invention;
- figure 3 shows a detail view of some components of the biomechatronic device for automated diagnosis of rheumatic diseases, according to the invention;

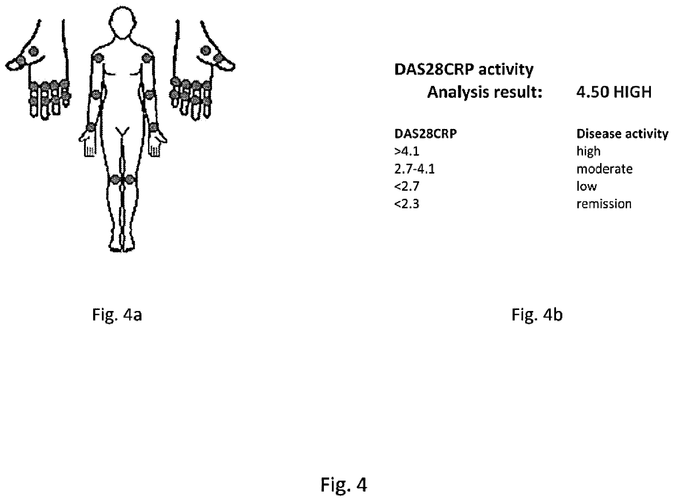

- figures 4a and 4b show, respectively, an anatomical map of articular joints of a human body to which the biomechatronic device for automated diagnosis of rheumatic diseases is able to be applied, and an output generated by the biomechatronic device, according to the invention.

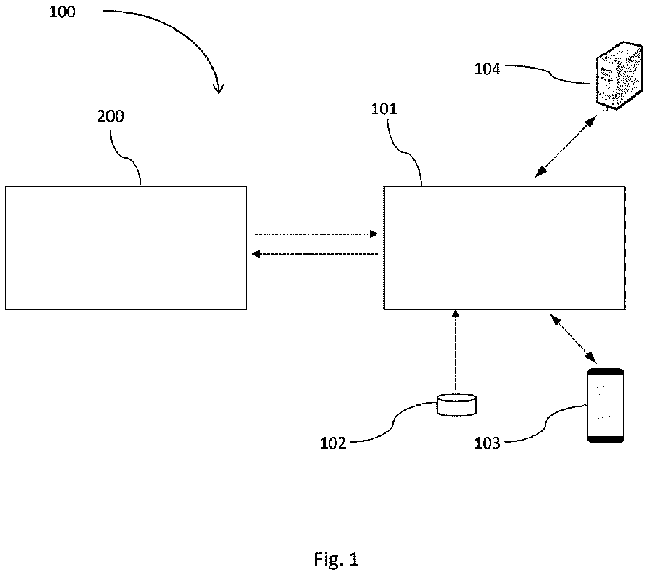

With reference to these figures and, in particular, to figure 1, a biomechatronic device for automated diagnosis of rheumatic diseases is shown, according to the invention.

In particular, the biomechatronic device 100 for automated diagnosis of rheumatic diseases comprises:
- at least one wearable element 200 comprising at least one actuator 100a configured to apply mechanical nociceptive stimuli to at least one articular joint of a user, and at least one force sensor 100c able to detect the intensity of the mechanical nociceptive stimuli applied by said at least one actuator 100a;
- an electrical air compressor able to generate a force through a pneumatic drive of the at least one actuator 100a;
- at least one transmission mechanical element 100b connected to the at least one actuator 100a and configured to transmit the force generated by the electrical air compressor to said at least one actuator 100a, said force corresponding to the mechanical nociceptive stimuli applied to at least one articular joint of the user;
- a button 102 configured to be actuated by the user, able to interrupt the application of the mechanical nociceptive stimuli and to report reaching the pain threshold.

According to an aspect of the invention, the biomechatronic device 100 comprises an electronic control unit (101, connected to the at least one wearable element 200, to the button 102 and to the electrical air compressor, comprising a monitoring and control module able to manage the mechanical nociceptive stimuli applied by the biomechatronic device 100.

According to an aspect of the invention, the electronic control unit 101 is able to acquire the instantaneous value of the mechanical nociceptive stimuli detected by the force sensor 100c, when the button 102 is actuated by the user to signal the perception of pain.

The biomechatronic device 100 may comprise a support structure 100d configured to house the at least one actuator 100a, the at least one transmission mechanical element 100b and the at least one force sensor 100c.

The support structure 100d may be configured for positioning the wearable element 200, in particular the actuator 100a, in correspondence with at least one articular joint of the user/patient.

In use, the wearable element 200 is an exoskeleton, made according to the state of the art of 'soft robotics', that healthcare professionals, more specifically rheumatologists, apply to an anatomical portion, and in particular to one or more articular joints, of the user/patient, for example on hands, elbows, knees, shoulders.

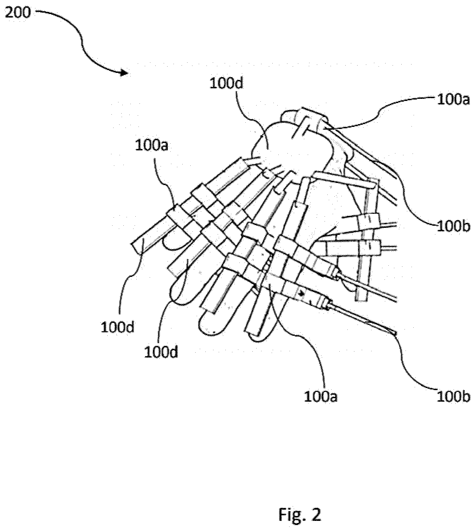

In a particular case better shown in figure 2, the wearable element 200 is applied to a hand and a wrist of the user.

With reference to the exoskeleton to be applied to a user's hand, the actuator 100a may be a deformable ring able to enclose a finger or a wrist of the user near an articular joint.

The at least one transmission mechanical element 100b may be a Bowden cable comprising internally a tie-rod 100ba.

The at least one force sensor 100c may be a piezoresistive force sensor placed in an inner portion of the actuator 100a in correspondence with the articular joint.

The actuator 100a and the support structure 100d may have adjustable dimensions.

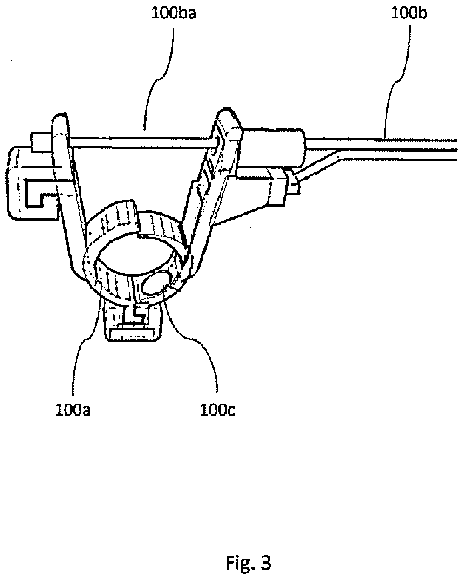

The actuator 100a may have dimensions able to be adjusted by applying half-rings of variable size, that is, semi-cylindrical elements fixed together at a respective end, as shown in figure 3.

The actuator 100a may be made by means of a deformable elastic structure, a soft actuator, pneumatically powered through a pneumatic subsystem, i.e. through compressed air delivered by the electric air compressor, not shown in the figure.

The support structure 100d may have dimensions adjustable by means of the variable positioning of the actuator 100a over said support structure 100d through clamped clamp joints.

Advantageously, the possibility of adjusting the dimensions of the support structure 100d allows to adapt the biomechatronic device 100 to the different anatomical conformations of several users.

Advantageously, the elasticity of the actuators 100a used for applying the mechanical nociceptive stimuli to the articular joints of the fingers of the user, in the particular case of the wearable element 200 applied to a hand, allows to use a single-effect actuator 100a, specifically a pneumatic muscle actuator.

In use, the initial position of the biomechatronic device 100 is reacquired, following the active phase of pneumatic compression, thanks to the elastic return ensured by the deformable structure of the actuators 100a.

Advantageously, the biomechatronic device 100 allows to reproduce the characteristics of multi-channel and distributed measurement, typical of the manual procedure performed by rheumatologists.

As previously described, the biomechatronic device 100 comprises an electronic control unit 101, schematized in Figure 1, connected to the wearable element 200 and to the electric air compressor, comprising the monitoring and control module able to manage the mechanical nociceptive stimuli and the entire biomechatronic device 100.

According to a preferred embodiment, the wearable element 200 comprises at least one actuator 100a for each finger of a user's hand, each actuator 100a being associable with an independent stimulation channel, i.e. an articular joint, with trends and intensity values of the force applied able to be modulated, independently for each actuator 100a, or also in a coordinated manner on different joints.

According to another embodiment, shown in figure 2, each finger of a user's hand wears a pair of actuators 100a, connected to a support rod, so that the wearable element 200 comprises ten actuators 100a which apply the forces related to the mechanical nociceptive stimuli, the intensities of which are regulated by the monitoring and control module. Each support rod is connected to a support plate, part of the support structure 100d and placed on the back of the user's hand.

The biomechatronic device 100 may comprise also an actuator 100a, able to be positioned in correspondence with the wrist of the user, that applies a mechanical nociceptive stimulus similar to that of the actuator 100a applied to a finger of the same user.

The actuator 100a may be configured to apply to an articular joint of the user a mechanical nociceptive stimulus in terms of a force that increases linearly over time or, secondarily, that can be modulated according to a trend programmable from time to time on the basis of specific needs concerning the diagnosis of pathologies.

The monitoring and control module, for example a software module including routines for programming and scheduling the mechanical nociceptive stimuli, that includes also a management firmware able to manage the electronics of the biomechatronic device 100, may comprise also a safety subsystem able to reduce the force applied by the actuator 100a, i.e. reducing the mechanical nociceptive stimuli applied, in case of absence of the power supply of the biomechatronic device 100, or in case the mechanical nociceptive stimuli, related to the force applied to each articular joint and detected through the force sensors 100c, exceeds a safety threshold value suitably calibrated on the biomechatronic device 100.

In use, the configuration of the monitoring and control module includes, in particular, the programming of the sequence of the multi-channel mechanical nociceptive stimuli, and the real-time visualization of the diagnostic parameters, said parameters being an output of the biomechatronic device 100. Such configuration functions are supplied to healthcare professionals through an HMI (Human Machine Interface) that resides on web pages preloaded on the electronic control unit 101.

Advantageously, the visualization of the operating parameters and the configuration of the biomechatronic device 100 for the automated diagnosis of rheumatic diseases, through the HMI interface, allow the healthcare professionals to operate comfortably through a user-friendly computer tool, as well as to view any on-screen alerts concerning failures of the biomechatronic device 100.

In particular, the aforementioned safety subsystem included in the monitoring and control module is able to interrupt the application of the compression force applied to a specific articular joint in the event of a power failure, or in the event that the applied force exceeds a predetermined threshold value.

Advantageously, the safety subsystem ensures the safety of the user in case of sudden power failure, or in case of exceeding the safety threshold of the maximum force applied to the single articular joints, avoiding the persistence of the pneumatic stimuli performed by the actuators 100a, and their releasing form the joints under diagnostic investigation.

The electronic control unit 101 may comprise a wireless module able to connect the biomechatronic device 100 to a data transmission network.

Advantageously, the wireless module, for example a Bluetooth LE module, allows to manage the monitoring and control module through a specific 'app' installed on a smartphone or tablet 103 supplied to healthcare professionals and/or bioengineers.

The electronic control unit 101 may comprise a wired or wireless network module, for example a Wi-Fi or a Ethernet port.

The use of the wired or wireless network module allows to send to a local or cloud server 104 data detected by the biomechatronic device 100, in particular the applied mechanical nociceptive stimuli, their instantaneous value upon activation of the button 102, i.e. in correspondence with the reaching of the pain threshold of the user, date and time of the measurement and the data associated with the user on whom the measurement is being carried out.

The electronic control unit 101 may comprise a programmable embedded electronic control board, for example an Arduino PCB, a Raspberry PI or another embedded mini PC based on a microcontroller unit.

A further advantage of the biomechatronic device 100 is that, by means of the Wi-Fi or Ethernet network port, said biomechatronic device 100 can be placed into a network, either local or remote, useful for integrating it into a telemedicine service.

In use therefore, advantageously, the sharing of an electronic health record, preferably residing on the local or cloud server 104, allows to effectively follow the temporal evolution of rheumatic disease for the individual user.

In the current diagnostic and therapeutic practice in rheumatology, the pain threshold is one of the parameters on which the assessment of the severity, together with the estimate of the corresponding evolution, of rheumatoid arthritis, is based. The current pain threshold assessment protocol in rheumatic patients lacks objectivity, since it depends on the individual perception and the close dependence on the specific health professional who performs the diagnostic tests. The classification of the level of severity of rheumatic diseases is carried out by reporting on an anatomical map, in the positions corresponding to any single articular joint of the user/patient, manually stimulated by the health professionals, specifically the rheumatologists, the levels of perception of pain concerning any aforementioned joint. The intensity of pain is reported to the rheumatologist by the patient using a numerical value included in a scale, the VAS (Visual Analogic Scale), whose values are comprised between 0 and 100.

The algorithm developed by the Applicant, running like other software routines included in the monitoring and control module in the electronic control unit 101, allows the automatic assessment of the severity of the rheumatic pathology. All this is achieved by aggregating, in a single index, the evaluation of the severity of the rheumatic disease, starting from the individual pain threshold measures resulting from the mechanical nociceptive force applied and measured on the single articular joints by means of the biomechatronic device 100.

In more detail, the biomechatronic device 100 allows an estimate of the severity of the disease as an output of the algorithm based on the measurements of nociceptive thresholds obtained automatically and in a distributed way by the individual channels, i.e. each single actuator 100a and each single force sensor 100c, that convey to the electronic control unit 101 the intensity of the mechanical nociceptive stimuli applied to the articular joints. In use, as mentioned, the user reports the sensation of pain by mechanically acting on the button 102 connected to the electronic control unit 101, which acquires, by sampling and saving it, the intensity of the corresponding mechanical nociceptive stimulus by means of the force sensors 100c.

In summary, the biomechatronic device 100 ensures, in an automatic way and according to the specifications of a software logic executed by the embedded electronic control board, the sequentially programmed generation and application of mechanical nociceptive stimuli on several articular joints of the user, the acquisition of pain thresholds related to such stimuli, and the evaluation of the severity index of a related rheumatic disease.

Advantageously, the biomechatronic device 100 allows to automate the diagnostic protocol in a multi-channel and objective mode, regardless of the operating modes of the specific rheumatologist.

According to the present disclosure a method of use of the biomechatronic device 100 described above is also provided, method comprising the steps:
- of applying at least one wearable element (200) comprised in the biomechatronic device (100) to a portion of the body of a user, said wearable element (200) comprising actuators able to apply mechanical nociceptive stimuli to at least one articular joint of the user, and said force sensors being able to detect the intensity of the mechanical nociceptive stimuli;
- of scheduling and managing a sequence of mechanical nociceptive stimuli by means of a monitoring and control module included in an electronic control unit connected to the wearable element (200), to an electrical air compressor configured to pneumatically drive the actuators by means of at least one transmission mechanical element connected to the wearable element (200), and to a button;
- of showing in real-time and on an anatomical map showing the articular joints of the user the intensities of the mechanical nociceptive stimuli, sampled and acquired by the electronic control unit at an instant corresponding to the time of the pressure applied by the user to the button;
- of evaluating the level of the severity of a rheumatic pathology based on pain thresholds following the application of the mechanical nociceptive stimuli, said pain thresholds being acquired by the electronic control unit.

Advantageously, the method of use of the biomechatronic device 100 allows to perform measurements without the intervention of the user, except for the pressure of the button, since all the steps concerning such measurements are actively carried out by means of the hardware and the logics of the biomechatronic device 100.

Therefore, the biomechatronic device for automated diagnosis of rheumatic diseases according to the disclosure allows the reconstruction of the anatomical map of the intensity levels of the pain threshold concerning the articular joints of the user.

Another advantage of the biomechatronic device for automated diagnosis of rheumatic diseases according to the disclosure is that it allows to keep in the desired anatomical positions, i.e. the articular joints, the actuators and the sensors used to automate the measurement and ensure that the anatomical points, displayed on the anatomical map related to the pain intensity, match the actual positions on the articular joints of the patient, with the necessary characteristics of mechanical stability and precision of the stimulus and of the measurement. This scope, as described, is achieved thanks to a wearable robotic hardware, similar to an exoskeleton, which guarantees, in a mechanically stable way, the distribution of the stimulation and sampling points of the nociceptive signals provided on the anatomical map related to the traditional protocol, while ensuring the necessary measurement precision and transmission accuracy of the mechanical nociceptive stimuli.

Another advantage of the biomechatronic device for automated diagnosis of rheumatic diseases according to the disclosure is that it ensures an objective benchmark for assessing the pain threshold and estimating the severity of the disease, remedying the lack of repeatability of the diagnostic protocols from which the known technical solutions suffer.

A further advantage of the biomechatronic device for automated diagnosis of rheumatic diseases according to the disclosure is that its use is able to be applied to scientific research, to experimental and clinical treatments of rheumatic diseases, to the pain treatment, to the rehabilitation and to the formulation of analgesic drugs.

In addition, the biomechatronic device for automated diagnosis of rheumatic diseases according to the disclosure is compatible with the diagnosis that makes use of the functional MRI, a known biomedical imaging technique useful for evaluating the functionality of an organ or apparatus.

A further advantage of the biomechatronic device for automated diagnosis of rheumatic diseases according to the disclosure is that it is safe.

Furthermore, the biomechatronic device for automated diagnosis of rheumatic diseases according to the disclosure is easy to use.

Finally, the biomechatronic device for automated diagnosis of rheumatic diseases according to the disclosure is inexpensive and involves a reduced complexity concerning the prototyping and industrialization phases.

It is finally clear that the biomechatronic device for automated diagnosis of rheumatic diseases described and illustrated herein can be subject to modifications and variations without departing from the protective scope of the present invention, as defined in the appended claims.

## Claims

1. Biomechatronic device (100) for automated diagnosis of rheumatic diseases, comprising:
- at least one wearable element (200) comprising at least one actuator (100a) configured to apply mechanical nociceptive stimuli to at least one articular joint of a user, and at least one force sensor (100c);
- an electrical air compressor able to generate a force through a pneumatic drive of the at least one actuator (100a);
- at least one transmission mechanical element (100b) connected to the at least one actuator (100a) and configured to transmit the force generated by the electrical air compressor to said at least one actuator (100a), said force corresponding to the mechanical nociceptive stimuli applied to at least one articular joint of the user;
- a button (102);
- an electronic control unit (101) connected to the at least one wearable element (200), to the button (102) and to the electrical air compressor;
wherein: 1) the at least one force sensor (100c) is able to detect the intensity of the mechanical nociceptive stimuli applied by said at least one actuator (100a), 2) the button (102) is configured to be actuated by the user and is able to interrupt the application of the mechanical nociceptive stimuli and to report reaching the pain threshold, and 3) the electronic control unit (101) comprises a monitoring and control module able to manage the mechanical nociceptive stimuli applied by the biomechatronic device (100), said electronic control unit (101) being able to acquire the instantaneous value of the mechanical nociceptive stimuli detected by the at least one force sensor (100c) when the button (102) is actuated by the user.

2. Biomechatronic device (100) according to claim 1, **characterized in** comprising a support structure (100d) configured to house the at least one actuator (100a), the at least one transmission mechanical element (100b) and the at least one force sensor (100c), said support structure (100d) being able to position the wearable element (200) near at least one articular joint of the user.

3. Biomechatronic device (100) according to claim 1, **characterized in that** the at least one actuator (100a) is at least one deformable ring configured to enclose a finger or a wrist of the user near an articular joint.

4. Biomechatronic device (100) according to claim 1, **characterized in that** the at least one transmission mechanical element (100b) is a Bowden cable comprising internally a tie-rod (100ba).

5. Biomechatronic device (100) according to claim 1, **characterized in that** the at least one force sensor (100c) is a piezoresistive force sensor placed in an inner portion of the at least one actuator (100a) in correspondence with the articular joint.

6. Biomechatronic device (100) according to claim 1, **characterized in that** the at least one actuator (100a) has dimensions adjustable by applying half cylinders having a variable size.

7. Biomechatronic device (100) according to claim 1, **characterized in that** the at least one actuator (100a) is made by means of a deformable elastic structure powered by a pneumatic subsystem.

8. Biomechatronic device (100) according to claim 1, **characterized in that** the support structure (100d) has dimensions able to be adjusted by means of the variable positioning of the at least one actuator (100a) over said support structure (100d) through clamped joints.

9. Biomechatronic device (100) according to one of the previous claims, **characterized in that** said electronic control unit (101) comprises a monitoring and control module comprising a safety subsystem able to reduce the applied force by reducing the mechanical nociceptive stimuli in case of absence of the power supply of the biomechatronic device (100), or in case the mechanical nociceptive stimuli, related to the force applied to each articular joint and detected by means of the force sensors (100c) exceeds a safety threshold value, said safety threshold value being calibrated on the biomechatronic device (100).

10. Biomechatronic device (100) according to claim 1, **characterized in that** the electronic control unit (101) comprises a wireless module able to connect the biomechatronic device (100) to a data transmission network.

11. Biomechatronic device (100) according to claim 1, **characterized in that** the electronic control unit (101) comprises a programmable embedded electronic control board.

12. Method of use of the biomechatronic device (100) according to one of the previous claims, **characterized in** comprising the steps:
- of applying at least one wearable element (200) comprised in the biomechatronic device (100) to a portion of the body of a user, said wearable element (200) comprising actuators able to apply mechanical nociceptive stimuli to at least one articular joint of the user, and said force sensors being able to detect the intensity of the mechanical nociceptive stimuli;
- of scheduling and managing a sequence of mechanical nociceptive stimuli by means of a monitoring and control module included in an electronic control unit connected to the wearable element (200), to an electrical air compressor configured to pneumatically drive the actuators by means of at least one transmission mechanical element connected to the wearable element (200), and to a button;
- of showing in real-time and on an anatomical map showing the articular joints of the user the intensities of the mechanical nociceptive stimuli, sampled and acquired by the electronic control unit at an instant corresponding to the time of the pressure applied by the user to the button.

## Patentansprüche

1. Biomechatronisches Gerät (100) für das automatisierte Diagnostizieren rheumatischer Erkrankungen, das Folgendes umfasst:
- mindestens ein tragbares Element (200) mit mindestens einem Aktuator (100a), der so konfiguriert ist, dass er mechanische nozizeptive Reize auf mindestens ein Gelenk des Benutzers ausübt, und mindestens einen Kraftsensor (100c);
- einen elektrischen Luftkompressor zum Erzeugen einer Kraftdurch einen pneumatischen Antrieb des mindestens einen Aktuators (100a);
- mindestens ein mechanisches Übertragungselement (100b), das mit dem mindestens einen Aktuator (100a) verbunden und so konfiguriert ist, dass es die von dem elektrischen Luftkompressor erzeugte Kraft auf den mindestens einen Aktuator (100a) überträgt, wobei die Kraft den mechanischen nozizeptiven Reizen entspricht, die auf mindestens ein Gelenk des Benutzers einwirken;
- eine Taste (102);
- eine elektronische Steuerungseinheit (101), die mit dem mindestens einen tragbaren Element (200), mit der Taste (102) und mit dem elektrischen Luftkompressor verbunden ist;
wobei:
1) der mindestens eine Kraftsensor (100c) die Intensität der vom mindestens einen Stellglied (100a) ausgeübten mechanischen nozizeptiven Reize erfassen kann,
2) die Taste (102) vom Benutzer betätigt werden und das Ausüben der mechanischen nozizeptiven Reize unterbrechen sowie das Erreichen der Schmerzschwelle melden kann, und
3) die elektronische Steuerungseinheit (101) ein Überwachungs- und Steuerungsmodul umfasst, das die vom biomechatronischen Gerät (100) ausgeübten mechanischen nozizeptiven Reize steuern kann, wobei die elektronische Steuerungseinheit (101) den vom mindestens einen Kraftsensor (100c) erfassten Momentanwert der mechanischen nozizeptiven Reize erfassen kann, wenn die Taste (102) durch vom Benutzer betätigt wird.

2. Biomechatronisches Gerät (100) gemäß Anspruch 1, **dadurch gekennzeichnet, dass** dieses eine Trägerstruktur (100d) umfasst, die den mindestens einen Aktuator (100a), das mindestens eine mechanische Übertragungselement (100b) und den mindestens einen Kraftsensor (100c) aufnimmt, wobei die Trägerstruktur (100d) das tragbare Element (200) in der Nähe mindestens eines Gelenks des Benutzers positionieren kann.

3. Biomechatronisches Gerät (100) gemäß Anspruch 1, **dadurch gekennzeichnet, dass** es sich beim mindestens einen Aktuator (100a) um mindestens einen verformbaren Ring handelt, der in der Nähe eines Gelenks einen Finger oder ein Handgelenk des Benutzers umschließt.

4. Biomechatronisches Gerät (100) gemäß Anspruch 1, **dadurch gekennzeichnet, dass** das es sich bei mindestens einem mechanischen Übertragungselement (100b) um einen Bowdenzug handelt, der im Inneren eine Zugstange (100ba) aufweist.

5. Biomechatronisches Gerät (100) gemäß Anspruch 1, **dadurch gekennzeichnet, dass** es sich beim mindestens einen Kraftsensor (100c) um einen piezoresistiven Kraftsensor handelt, der ausgerichtet am Gelenk im Inneren des mindestens einen Aktuators (100a) angeordnet ist.

6. Biomechatronisches Gerät (100) gemäß Anspruch 1, **dadurch gekennzeichnet, dass** der mindestens eine Aktuator (100a) Abmessungen aufweist, die durch das Anbringen von Halbzylindern mit variabler Größe eingestellt werden.

7. Biomechatronisches Gerät (100) gemäß Anspruch 1, **dadurch gekennzeichnet, dass** der mindestens eine Aktuator (100a) durch eine verformbare elastische Struktur gebildet wird, die von einem pneumatischen Untersystem angetrieben wird.

8. Biomechatronisches Gerät (100) gemäß Anspruch 1, **dadurch gekennzeichnet, dass** die Trägerstruktur (100d) Abmessungen aufweist, die durch die variable Positionierung des mindestens einen Aktuators (100a) über der Trägerstruktur (100d) mithilfe von Klemmverbindungen eingestellt werden können.

9. Biomechatronisches Gerät (100) gemäß einem der vorherigen Ansprüche, **dadurch gekennzeichnet, dass** die elektronische Steuerungseinheit (101) ein Überwachungs- und Steuerungsmodul umfasst, das ein Sicherheitsuntersystem umfasst, das die angewendete Kraft verringern kann, indem es im Falle eines Ausfalls der Stromversorgung des biomechatronischen Geräts (100)oder im Falle von mechanischen nozizeptiven Reizen, deren auf die einzelnen Gelenke ausgeübte und von den Kraftsensoren (100c) erfasste Kraft einen Sicherheitsschwellenwert überschreitet, die mechanischen nozizeptiven Reize reduziert, wobei dieser Sicherheitsschwellenwert auf dem biomechatronischen Gerät (100) kalibriert wird.

10. Biomechatronisches Gerät (100) gemäß Anspruch 1, **dadurch gekennzeichnet, dass** die elektronische Steuerungseinheit (101) ein drahtloses Modul umfasst, das das biomechatronische Gerät (100) mit einem Datenübertragungsnetzverbinden kann.

11. Biomechatronisches Gerät (100) gemäß Anspruch 1, **dadurch gekennzeichnet, dass** die elektronische Steuerungseinheit (101) eine programmierbare integrierte elektronische Steuerungsplatine umfasst.

12. Methode zum Verwenden des biomechatronischen Geräts (100) gemäß einem der vorherigen Ansprüche, **dadurch gekennzeichnet, dass** diese folgende Schritte umfasst:
- Anlegen mindestens eines im biomechatronischen Gerät (100) enthaltenen tragbaren Elements (200) an einen Teil des Körpers eines Benutzers, wobei das tragbare Element (200) Aktuatoren umfasst, die mechanische nozizeptive Reize auf mindestens ein Gelenk des Benutzers ausüben können, und wobei die Kraftsensoren die Intensität der mechanischen nozizeptiven Reize erfassen können;
- Planen und Verwalten einer Sequenz von mechanischen nozizeptiven Reizen mithilfe eines Überwachungs- und Steuerungsmoduls, das in einer mit dem tragbaren Element (200) verbundenen elektronischen Steuerungseinheit enthalten ist, und das über einen elektrischen Luftkompressor verfügt, der die Aktuatoren mithilfe mindestens eines mit dem tragbaren Element (200) verbundenen mechanischen Übertragungselements pneumatisch antreibt, und das zudem eine Taste umfasst;
- in Echtzeit und auf einer anatomischen Karte mit den Gelenken des Benutzers Anzeigen der Intensitäten der mechanischen nozizeptiven Reize, die von der elektronischen Steuereinheit zu einem Zeitpunkt abgetastet und erfasst werden, der dem Zeitpunkt des Drucks entspricht, den der Benutzer auf die Taste ausübt.

## Revendications

1. Dispositif biomécatronique (100) pour le diagnostic automatisé de maladies rhumatismales, comprenant
- au moins un élément portable (200) comprenant au moins un actionneur (100a) configuré pour appliquer des stimuli mécaniques nociceptifs à au moins une articulation d'un utilisateur, et au moins un capteur de force (100c) ;
- un compresseur d'air électrique capable de générer une force par l'intermédiaire d'un entraînement pneumatique de l'au moins un actionneur (100a);
- au moins un élément mécanique de transmission (100b) relié à l'au moins un actionneur (100a) et configuré pour transmettre la force générée par le compresseur d'air électrique audit au moins un actionneur (100a), ladite force correspondant aux stimuli mécaniques nociceptifs appliqués à au moins une articulation de l'utilisateur ;
- un bouton (102) ;
- une unité de commande électronique (101) connectée à l'au moins un élément portable (200), au bouton (102) et au compresseur d'air électrique ;
dans lequel :
1) le au moins un capteur de force (100c) est capable de détecter l'intensité des stimuli mécaniques nociceptifs appliqués par ledit au moins un actionneur (100a),
2) le bouton (102) est configuré pour être actionné par l'utilisateur et est capable d'interrompre l'application des stimuli mécaniques nociceptifs et de signaler l'atteinte du seuil de douleur, et
3)l'unité de commande électronique (101) comprend un module de surveillance et de commande capable de gérer les stimuli mécaniques nociceptifs appliqués par le dispositif biomécatronique (100), ladite unité de commande électronique (101) étant capable d'acquérir la valeur instantanée des stimuli mécaniques nociceptifs détectés par le au moins un capteur de force (100c) lorsque le bouton (102) est actionné par l'utilisateur.

2. Dispositif biomécatronique (100) selon la revendication 1, **caractérisé en ce qu'**il comprend une structure de support (100d) configurée pour loger le au moins un actionneur (100a), le au moins un élément mécanique de transmission (100b) et le au moins un capteur de force (100c), ladite structure de support (100d) étant capable de positionner l'élément portable (200) près d'au moins une articulation de l'utilisateur.

3. Dispositif biomécatronique (100) selon la revendication 1, **caractérisé en ce que** le au moins un actionneur (100a) est au moins un anneau déformable configuré pour entourer un doigt ou un poignet de l'utilisateur près d'une articulation.

4. Dispositif biomécatronique (100) selon la revendication 1, **caractérisé en ce que** le au moins un élément mécanique de transmission (100b) est un câble Bowden comprenant en interne une barre de liaison (100ba).

5. Dispositif biomécatronique (100) selon la revendication 1, **caractérisé en ce que** le au moins un capteur de force (100c) est un capteur de force piézorésistif placé dans une partie interne du au moins un actionneur (100a) en correspondance avec l'articulation.

6. Dispositif biomécatronique (100) selon la revendication 1, **caractérisé en ce que** le au moins un actionneur (100a) a des dimensions réglables en appliquant des demi-cylindres ayant une taille variable.

7. Dispositif biomécatronique (100) selon la revendication 1, **caractérisé en ce que** le au moins un actionneur (100a) est réalisé au moyen d'une structure élastique déformable alimentée par un sous-système pneumatique.

8. Dispositif biomécatronique (100) selon la revendication 1, **caractérisé en ce que** la structure de support (100d) a des dimensions pouvant être ajustées au moyen du positionnement variable de l'au moins un actionneur (100a) sur ladite structure de support (100d) par l'intermédiaire de joints serrés.

9. Dispositif biomécatronique (100) selon l'une des revendications précédentes, **caractérisé en ce que** ladite unité de commande électronique (101) comprend un module de surveillance et de commande comprenant un sous-système de sécurité capable de réduire la force appliquée en réduisant les stimuli mécaniques nociceptifs en cas d'absence de l'alimentation électrique du dispositif biomécatronique (100)ou dans le cas où les stimuli mécaniques nociceptifs, liés à la force appliquée à chaque articulation et détectés au moyen des capteurs de force (100c), dépassent une valeur limite de sécurité, ladite valeur limite de sécurité étant calibrée sur le dispositif biomécatronique (100).

10. Dispositif biomécatronique (100) selon la revendication 1, **caractérisé en ce que** l'unité de commande électronique (101) comprend un module sans fil capable de connecter le dispositif biomécatronique (100) à un réseau de transmission de données.

11. Dispositif biomécatronique (100) selon la revendication 1, **caractérisé en ce que** l'unité de commande électronique (101) comprend une carte de commande électronique intégrée programmable.

12. Procédé d'utilisation du dispositif biomécatronique (100) selon l'une des revendications précédentes, **caractérisé en ce qu'**il comprend les étapes consistant à :
- appliquer au moins un élément portable (200) compris dans le dispositif biomécatronique (100) à une partie du corps d'un utilisateur, ledit élément portable (200) comprenant des actionneurs capables d'appliquer des stimuli mécaniques nociceptifs à au moins une articulation de l'utilisateur, et lesdits capteurs de force étant capables de détecter l'intensité des stimuli mécaniques nociceptifs ;
- programmer et gérer une séquence de stimuli mécaniques nociceptifs au moyen d'un module de surveillance et de commande inclus dans une unité de commande électronique connectée à l'élément portable (200), à un compresseur d'air électrique configuré pour entraîner par voie pneumatique les actionneurs au moyen d'au moins un élément mécanique de transmission connecté à l'élément portable (200), et à un bouton ;
- montrer en temps réel et sur une carte anatomique présentant les articulations de l'utilisateur les intensités des stimuli mécaniques nociceptifs, échantillonnés et acquis par l'unité de commande électronique à un instant correspondant au moment de la pression appliquée par l'utilisateur sur le bouton.
